Europäisches Patentamt

European Patent Office

Office européen des brevets

(11) Publication number: 0 317 278
A2

(12) # EUROPEAN PATENT APPLICATION

(21) Application number: 88310799.7

(22) Date of filing: 16.11.88

(51) Int. Cl.⁴: **C 07 K 13/00**
C 07 K 7/08, C 07 K 7/06,
A 61 K 37/02

(30) Priority: 17.11.87 US 121454

(43) Date of publication of application:
24.05.89 Bulletin 89/21

(84) Designated Contracting States:
AT BE CH DE ES FR GB GR IT LI LU NL SE

(71) Applicant: SCRIPPS CLINIC AND RESEARCH
FOUNDATION
10666 North Torrey Pines Road
La Jolla California 92037-1093 (US)

(72) Inventor: Zimmerman, Theodore S.
544 Bonair Street
La Jolla California 92037 (US)

Ruggeri, Zaverio M.
644 Bonair Street
La Jolla California 92037 (US)

Houghten, Richard A.
558 Ford Avenue
Solona Beach California 92075 (US)

Vincete, Vincete
4249 Nobel Drive
San Diego California 92122 (US)

Mohri, Hiroshi
7781 Camino Glorita
San Diego California 92037 (US)

(74) Representative: Jump, Timothy John Simon et al
VENNER, SHIPLEY & CO. 368 City Road
London EC1V 2QA (GB)

(54) **Proteolytic fragments and synthetic peptides that block the binding of von Willebrand factor to the platelet membrane glycoprotein Ib.**

(57) Peptides which inhibit the binding of von Willebrand factor to platelet membrane glycoprotein Ib and platelet membrane glycoprotein Ib related molecules or other cells and cell matrices. The method of preventing platelet aggregation or thrombosis by administering one of these peptides is also disclosed.

EP 0 317 278 A2

**Description**

# PROTEOLYTIC FRAGMENTS AND SYNTHETIC PEPTIDES THAT BLOCK THE BINDING OF VON WILLEBRAND FACTOR TO THE PLATELET MEMBRANE GLYCOPROTEIN Ib.

This invention relates to peptides which inhibit the binding of von Willebrand factor (vWF) to platelet membrane glycoprotein Ib (GPIb) and platelet membrane GPIb related molecules or other cells and cell matrices.

This invention also relates to the prevention of platelet aggregation.

This invention also relates to the prevention of thrombosis.

vWF participates in the early stages of hemostasis by binding to the platelet GPIb receptor. The importance of the interaction between vWF and GPIb is suggested by the bleeding diathesis of the Bernard-Soulier syndrome, a disorder characterized by the decreased quantities or abnormal function of GPIb. The GPIb acts as the receptor for vWF in mediating the adhesion of platelets to exposed subendothelial matrix, altered endothelial cells and thrombogenic surfaces in general, particularly under conditions of high wall shear stress (high flow rate in small vessels). Inhibition of vWF-GPIb interaction would thus be expected to result in the prevention of primary hemostasis and the induction of an anti-thrombotic state useful in prevention of diseases in which occlusion of blood vessels plays an important role. The proteolytic fragments of GPIb and peptides of this present invention have the ability to act as anti-thrombotic agents by their prevention of the binding of vWF to GPIb.

The present invention provides any peptide of a 45 kDa amino terminal tryptic fragment of glycocalicin selected from the following amino acid sequence:

```
        10        20        30        40        50        60
        |         |         |         |         |         |
HPICEVSKVASHLEVNCDKRNLTALPPDLPKDTTILHLSENLLYTFSLATLMPYTRLTQL

        70        80        90       100       110       120
        |         |         |         |         |         |
NLDRCELTKLQVDGTLPVLGTLDLSHNQLQSLPLLGQTLPALTVLDVSFNRLTSLPLGAL

       130       140       150       160       170       180
        |         |         |         |         |         |
RGLGELQELYLKGNELKTLPPGLLTPTPKLEKLSLANNNLTELPAGLLNGLENLDTLLLQ

       190       200       210       220       230       240
        |         |         |         |         |         |
ENSLYTIPKGFFGSHLLPFAFLHGNPWLCNCEILYFRRWLQDNAENVYVWKQGVDVKAMT

       250       260       270       280       290
        |         |         |         |         |
SNVASVQCDNSDKFPVYKYPGKGCPTLGDEGDTDLYDYYPEEDTEGDKVRATR
```

which inhibits the binding of von Willebrand factor to platelet membrane glycoprotein Ib and platelet membrane glycoprotein Ib related molecules or other cells and cell matrices.

The invention further provides any sequential subset of a 45 kDa amino terminal tryptic fragment of glycocalicin selected from the following amino acid sequence:

```
        10        20        30        40        50        60
         |         |         |         |         |         |
HPICEVSKVASHLEVNCDKRNLTALPPDLPKDTTILHLSENLLYTFSLATLMPYTRLTQL

        70        80        90       100       110       120
         |         |         |         |         |         |
NLDRCELTKLQVDGTLPVLGTLDLSHNQLQSLPLLGQTLPALTVLDVSFNRLTSLPLGAL

       130       140       150       160       170       180
         |         |         |         |         |         |
RGLGELQELYLKGNELKTLPPGLLTPTPKLEKLSLANNNLTELPAGLLNGLENLDTLLLQ

       190       200       210       220       230       240
         |         |         |         |         |         |
ENSLYTIPKGFFGSHLLPFAFLHGNPWLCNCEILYFRRWLQDNAENVYVWKQGVDVKAMT

       250       260       270       280       290
         |         |         |         |         |
SNVASVQCDNSDKFPVYKYPGKGCPTLGDEGDTDLYDYYPEEDTEGDKVRATR
```

which inhibits binding of von Willebrand factor to platelet membrane glycoprotein Ib and platelet membrane glycoprotein Ib related molecules or other cells and cell matrices.

The invention further provides a peptide which inhibits binding of von Willebrand factor to platelet membrane glycoprotein Ib and platelet membrane glycoprotein Ib related molecules or other cells and cell matrices selected from the group of peptides consisting of:

DKRNLTALPPDLPKDTT; NLTALPPDLPKDTTI; PPDLPKDTTILHLSE; PGLLTPTPKLEKLSL; KQGVDVKAMTSNVAS; GDTDLYDYYPEEDTE; EEDTEGDKVRATRTV; PPDLPKDTT; and EEDTE.

Additionally the present invention provides a peptide of any sequential subset of amino acids of a peptide and derivatives thereof which inhibits binding of von Willebrand factor to platelet membrane glycoprotein Ib and platelet membrane glycoprotein Ib related molecules or other cells and cell matrices.

The invention further comprises a peptide having the general formula (KR)n, wherein n=2-10 and $R_n$, wherein n=2-20 and any derivatives thereof which inhibits binding of von Willebrand factor to platelet membrane glycoprotein Ib and platelet membrane glycoprotein Ib related molecules or other cells and cell matrices.

The invention further comprises a method for inhibiting aggregation of platelets to each other with an effective amount of one of these peptides.

The invention further comprises a method for inhibiting thrombosis in a patient which comprises administering to said patient an effective amount of one of these peptides.

GPIb is a two-chain molecule composed of a heavy (alpha) chain of approximately 140 kDa molecular mass linked by disulfide bonds to a light (beta) chain of approximate molecular mass 22 kDa. GPIb is an integral membrane protein and both the alpha- and beta- chains have transmembrane domains. Proteolysis by an endogenous calcium-dependent platelet protease generates a proteolytic fragment of the alpha-chain known as glycocalicin. This fragment originates from the extracellular domain of the alpha-chain and is water soluble. Thus, it is released after cleavage from the parent molecule.

The following description provides details of the manner in which the embodiments of the present invention may be made and used. This description, while exemplary of the present invention, is not to be construed as specifically limiting the invention and such variations which would be within the purview of one skilled in this art are to be considered to fall within the scope of this invention.

We have purified glycocalicin with a two-step procedure based on 1) affinity chromatography using wheat germ agglutinin insolubilized onto Sepharose beads; and 2) subsequent immunoaffinity chromatography using a monoclonal antibody (LJ-P3) directed against glycocalicin and insolubilized onto Sepharose beads.

Outdated platelet concentrates were used as starting material for the purification of glycocalicin. Plasma components were eliminated by sedimenting the platelets at 2,300 g for 25 minutes at room temperature (22-25°C), removing the supernatant, and resuspending the platelet pellet in a buffer composed of 10 mM Tris base and 150 mM NaCl, adjusted to pH 7.4 with HCl (Tris-buffered saline; TBS), and containing 2 mM EDTA. This procedure was repeated twice. After the first wash, the suspension was centrifuged at 600 g for 1 minute and the pellet containing most of the contaminating red cells was discarded before continuing with the washing procedure. After the last centrifugation, the platelets were resuspended in TBS containing 2mM $CaCl_2$ and 0.1 mM phenylmethylsulfonyl fluoride (PMSF). They were then disrupted by sonication (three pulses of 15 seconds each at approximately 100 watts, with the platelet suspension kept on ice). The suspension was then left for three hours at room temperature and for 16-18 hours at 4°C, always with continuous stirring. Following this, the particulated material in the suspension was removed by centrifugation at 100,000 g for 20 minutes at 12°C. The clear supernatant was applied onto a column (2.6 cm in diameter and 11 cm high) of wheat germ agglutinin bound to Sepharose beads activated with cyanogen bromide and equilibrated with TBS containing 1 mM EDTA, 0.1 mM PMSF, and 0.02% sodium azide. The column was washed with a volume of buffer corresponding to twice the volume of beads before eluting bound proteins with 100 mM N-acetyl glucosamine added to the same buffer. The whole procedure was performed at room temperature. The eluted material was immediately applied onto a monoclonal antibody column (5 cm in diameter and 2.5 cm high) consisting of purified IgG bound to Sepharose beads activated with cyanogen bromide. The monoclonal antibody used, designated LJ-P3, is specific for the glycocalicin portion of GPIb; its preparation, characterization, and purification are described in Handa et al., J. Biol. Chem., 261, 12579-12585, 1986. The column was equilibrated with a buffer composed of 100 mM Tris base, 500 mM $LiCl_2$, 1 mM EDTA, 0.1 mM PMSF, 0.02% sodium azide, adjusted to pH 7.4 with HCl. The column was washed with a volume of buffer corresponding to three times the volume of beads. Bound glycocalicin was eluted with 70-80 ml of 50 mM diethylamine containing 1 mM EDTA and 0.1 mM PMSF. During this step, the flow rate through the column was regulated so that elution was complete in 20-25 minutes. The whole procedure was performed at room temperature. The eluted glycocalicin was collected in 6 g of glycine to neutralize the high pH of diethylamine. The purified material was dialyzed extensively against TBS, concentrated with Aquacide, and again dialyzed with TBS. Purified glycocalicin was stored in aliquots at -70°C.

Purified glycocalicin was digested with trypsin pretreated with N-tosyl-L-phenylalanine chloromethylketone. The enzyme substrate ratio was 1:200 and the reaction was allowed to proceed for 16-18 hours at 37°C. At the end of the incubation, trypsin activity was inhibited with a two-fold molar excess of (p-amidinophenyl)methanesulfonyl fluoride. The 45 kDa fragment of glycocalicin generated by trypsin digestion was purified by high-pressure liquid gel permeation chromatography using one GF 450 and two GF 250 Du Pont Zorbax columns (9.4 mm in diameter by 25 cm in length) mounted in series. The columns were equilibrated with 200 mM $(NH_4)_2HPO_4$, pH 7, and the flow rate was 1 ml/minute. The procedure was performed at room temperature. The 45 kDa fragment eluted as a sharp peak that was collected, concentrated with Aquacide, dialyzed extensively with TBS, and then stored in aliquots at -70 °C until used.

We have used the purified glycocalicin to demonstrate that this proteolytic fragment of the alpha-chain of GPIb can inhibit the binding of vWF to intact platelets. The assay system is based on the use of $^{125}$I-labeled vWF and fresh or formalin-fixed platelets; ristocetin was used to induce the binding of vWF to GPIb. After incubation for 30 min at 37°C without stirring, separation of platelet-bound from free ligand was achieved by centrifugation through 20% sucrose in Tyrode buffer, followed by measurement of the bound radioactivity as described in Ruggeri et al., J. Clin Invest., 72, 1-12 (1983). Nonspecific binding was evaluated for selected points by measuring the binding in the presence of a 40-fold excess of unlabeled vWF. Binding isotherms were evaluated by Scatchard-type analysis to determine binding parameters (including the estimate of nonspecific binding) using the computer-assisted program Ligand as described in Munson, Methods Enzymol., 92, 542-576 (1983).

Glycocalicin at final concentrations in excess of 1 mg/ml can block the binding of $^{125}$I-labeled vWF to intact GPIb completely; the concentration necessary to inhibit 50% of the binding (denoted as the $IC_{50}$ value) was found to average 150 ug/ml using seven different glycocalicin preparations. Subsequently all the intra-chain disulfide bonds present in glycocalicin have been reduced by treatment with a molar excess of dithiothreitol and the resulting sulfhydryl groups have been blocked by S-carboxyimidomethylation. The resulting reduced and alkylated glycocalicin was found to retain the property of blocking vWF binding to intact GPIb on platelets in the presence of ristocetin. Since the reduced and alkylated glycocalicin had lost its secondary structure dependent on intra-chain disulfide bonds, this experiment demonstrated that the function of interacting with vWF could be ascribed to specific regions within the primary structure of glycocalicin.

Two proteolytic fragments of glycocalicin have been produced by treatment of the native molecule with trypsin. Trypsin has been shown to previously cleave glycocalicin between residue Arg-290 and Ala-291 or Arg-293 and Thr-294 to generate two fragments, one of which has an apparent molecular mass of 45 kDa and extends from the amino terminal residue His-1 to Arg-290 or Arg-293; the other, with an apparent molecular mass of 84 kDa, is very rich in carbohydrate and represents the carboxyl terminal half of the molecule beginning at Ala-291 or Thr-294. The 45 kDa fragment consists of a single-chain species and a two-chain species. The latter is generated by an additional tryptic cleavage between residues Lys-237 and Ala-238 yielding two polypeptides or apparent molecular mass 35 kDa and 7 kDa, respectively, held together by disulfide bonds. The relative proportion of the one- and two-chain species depends on the extent of tryptic cleavage of glycocalicin. After digestion for 18 hours with an enzyme to substrate ratio of 1:200 (weight to weight), the two-chain species is the predominant one.

The 45 kDa tryptic fragment of glycocalicin has been purified under native conditions using high pressure liquid chromatography (abbreviated HPLC) and gel permeation columns that separate proteins on the basis of their molecular mass. Because of the conditions used for tryptic digestion, the 45 kDa fragment consisted essentially of the two-chain species. This purified proteolytic fragment of glycocalicin has been used to test its ability to block the binding of vWF to the GPIb of platelets. The 45 kDa fragment inhibited completely the ristocetin-mediated binding of vWF to platelets, i.e. to GPIb, with an $IC_{50}$ of approximately 3.5 uM.

In a similar experiment, glycocalicin has been digested with trypsin, then disulfide bonds have been reduced with dithiothreitol and the resulting sulfhydryl groups have been S-carboxyimidomethylated with iodoacetamide. The 35 kDa amino terminal fragment has then been purified by gel permeation HPLC and tested for its inhibitory effect on the binding of vWF to the GPIb of platelets. Its $IC_{50}$ was found to be similar to that of the parent unreduced 45 kDa fragment. In accordance with the results obtained with whole glycocalicin, these results confirm that the primary structure of the amino terminal region of glycocalicin contains vWF binding domain(s) whose function does not depend on maintenance of the native three dimensional conformation of the molecule.

Following these findings, overlapping peptides composed of 15 amino acid residues and representing the sequence of the entire 45 kDa amino terminal fragment of glycocalicin have been synthesized. The following peptides have been found to inhibit the binding of vWF to the GPIb of platelets with $IC_{50}$ values of 0.5 mM or better (single-letter notation is used for the identification of amino acid residues): DKRNLTALPPDLKDTT; NLTALPPDLKDTTI; PPDLPKDTTILHLSE (these three peptides cover the sequence between residues Asp-18 and Glu-40 of glycocalicin); PGLLTPTPKLEKLSL (residues Pro-141 to Leu-155); KQGVDVKAMTSNVAS (residues Lys-231 to Ser-245); GDTDLYDYYPEEDTE; EEDTEGDKVRATRTV (these two peptides over the sequence between residues Gly-271 and Val-295). The results, therefore, clearly indicate the existence of multiple domains within the amino terminal region of glycocalicin that have functional relevance for vWF binding.

Shorter peptides with sequence corresponding to overlapping regions of the longer peptides exhibiting inhibitory activity also have been synthesized. Two of these shorter peptides were found to have inhibitory activity. Their sequence was: PPDLPKDTT (residues Pro-26 to Thr-34 of glycocalicin); and EEDTE (residues Glu-281 to Glu-285). These two peptides, when tested individually, had $IC_{50}$ values greater than 0.5 mM. When they were combined together at a concentration of 0.5 mM, however, they completely inhibited vWF binding to GPIb. This experiment demonstrates that different noncontiguous domains within the primary sequence of glycocalicin may participate concurrently in expressing vWF binding activity with higher activity. The sequence of the 45 kDa amino terminal tryptic fragment of glycocalicin, therefore, as well as subsets of it (as shown above) contains information useful for designing molecules capable of inhibiting the binding of vWF to the GPIb of platelets.

Peptides based on the amino acid sequence of the 45 kDa amino terminal tryptic fragment of glycocalicin and any sequential subset of it are synthesized as described by Houghton et al., Proc. Natl. Acad. Sci. USA, 82, 5135 (1985).

In the well known procedure for solid-phase synthesis of a peptide, the desired peptide is assembled starting from an insoluble support such as benzhydryl amine or chloromethylated resin (derived from cross-linked polystyrene, and available from chemical supply houses). The amino acid at the carboxy-terminal end of the desired peptide, carrying protecting groups on the alpha-amino nitrogen and on any other reactive sites, is attached to the resin from solution using known peptide coupling techniques. The protecting group on the alpha-amino group is removed (leaving other protecting group, if any, intact), and the next amino acid of the desired sequence (carrying suitable protecting groups) is attached, and so on. When the desired peptide has been completely built up, it is cleaved from the resin support, all protecting groups are removed, and the peptide is recovered. Examples of suitable protecting groups are: alpha-tert-butyloxycarbonyl for the alpha-amino-group; benzyl, 4-methoxybenzyl, or 4-methylbenzyl for the thiol group of cysteine, the beta-carboxylic acid group of aspartic acid, the gamma-carboxylic acid group of glutamic acid and the hydroxyl groups of serine, threonine, and tyrosine; benzyloxycarbonyl or a 2-chloro- or 3,4-dimethoxy-derivative thereof for the ring nitrogens of histidine and tryptophan and the epsilon-amino group of lysine; p-nitrophenyl for the amide nitrogens of asparagine and glutamine; and nitro or tosyl for the guanidine group of arginine.

For purposes of this disclosure, accepted short-hand designations of the amino acids have been used. A complete listing is provided herein below:

One and three-letter Amino Acid Abbreviations

| | | |
|---|---|---|
| A | Ala | Alanine |
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| B | Asx | Asp or Asn, not distinguished |
| Z | Glx | Glu or Gln, not distinguished |
| X | X | Undetermined or atypical amino acid |

The complete CDNA for GPIb has been determined by Lopez et al., Proc. Natl. Acad. Sci USA, 84, 5615-5619 (1987). With such information, a nucleotide sequence can be inserted into an appropriate vector for the expression of peptides from the 45 kDa fragment.

Peptides of the general formula $(KR)_n$, where n = 2-7, and the peptide $R_{11}$ inhibited the interaction of vWF with GPIb. Peptides of the general formula $R_nRGDV$ or $(KR)_nRGDV$ were previously demonstrated to block figrinogen binding to GPIIb/IIIa (U.S. Patent No. 4,683,291, "Platelet Binding Inhibitors,"). The latter are now shown to be equally effective in blocking vWF-GPIb interaction as their $(KR)_n$ analogues and therefore represent a class of bifunctional antiplatelet agents.

To test the inhibition of ristocetin-induced binding of vWF to platelets by peptides, formalin-fixed platelets were used at a final concentration of $1 \times 10^{11}$/liter. Peptides to be tested were then added at various concentrations. One third final volume of vWF deficient plasma was then added followed by $^{125}$I-vWF a final concentration of 5 micrograms/ml. Ristocetin was then added at a concentration of 1.0 mg/ml. After incubation for 30 minutes at room temperature bound and free ligand were separated by centrifuging 50 microliters of the mixture through 300 microliters of 20% sucrose at 12,000 x g for 4 minutes. The platelet pellet was then separated from the rest of the mixture to determine platelet-bound radioactivity. Nonspecific binding was defined as the residual binding of $^{125}$I-vWF in the presence of a 50-fold excess of unlabeled vWF but in the absence of any peptides.

Percent inhibition with a given peptide was calculated by dividing the specific cpm in the absence of peptide. The $IC_{50}$'s (concentration of peptide which inhibited binding by 50%) for the peptides tested were as follows:

| | |
|---|---|
| $(KR)_7$ | 9 and 15 micromolar (two experiments) |
| $(KR)_5$ | 13 micromolar |
| $(KR)_3$ | 120 micromolar |
| $(KR)_2$ | 200 micromolar |
| $(KR)_4GDV$ | 16 micromolar |
| $(R)_8GDV$ | 6 micromolar |
| YRGDV | > 600 micromolar (no inhibition seen) |

Complete inhibition was not seen with any of the peptides at the concentrations tested. To test the inhibitions of asialo-vWF binding to platelets, fresh platelets were used. These were prepared by drawing blood into 11 millimolar trisodium citrate and 2 millimolar EDTA. Platelet-rich plasma was then prepared by differential centrifugation. The platelet count was then adjusted to $1 \times 10^{11}$/liter. Peptides were then added to various concentrations and $^{125}$I-asialo-vWF was then added at a final concentration of 5 micrograms/ml. After incubation for 30 minutes at room temperature bound and free ligand were separated by centrifuging 50 microliters of the mixture through 300 microliters of 20% sucrose at 12,000 x g for 4 minutes. The platelet pellet was then separated from the rest of the mixture to determine platelet-bound radioactivity. Non-specific binding was defined as the residual binding of $^{125}$I-asialo-vWF in the presence of a 50-fold excess of unlabeled vWF but in the absence of any peptides.

Percent inhibition with a given peptide was calculated by dividing the specific cpm obtained when various concentrations of peptide were added by the specific cpm in the absence of peptide. The $IC_{50}$'s (concentration of peptide which inhibited binding by 50%) for the peptides tested were as follows:

| | |
|---|---|
| $(KR)_7$ | 1.5 micromolar |
| $(KR)_5$ | 1.7 micromolar |
| $(KR)_3$ | 23 micromolar |
| $(KR)_4GDV$ | 15 micromolar |
| $(R)_8GDV$ | 3.5 micromolar |
| $(R)_{11}$ | 7 micromolar |

Complete inhibition was seen at the following concentrations:

| | |
|---|---|
| $(KR)_7$ | 12 and 15 micromolar |
| $(KR)_5$ | 6 and 7 micromolar |
| $(KR)_3$ | 60 and 120 micromolar |
| $(KR)_4GDV$ | 44 micromolar |
| $(R)_8GDV$ | 24 micromolar |

Inhibition of ristocetin-induced platelet aggregation by peptides was evaluated using washed platelets. The platelets, prepared as described in Trapani-Lombardo et al. J. Clin. Invest., 76, 1950-1958 (1985), were adjusted to a final concentration of $3 \times 10^{11}$. Peptides, at varying concentrations, and purified vWF, at a final concentration of 0.8 micrograms/ml, were incubated with the platelets for 5 minutes at 37°C. Ristocetin was then added at a final concentration of 1.0 mg/ml. Reaction mixtures were prepared in siliconized glass cuvettes and then placed in a Lumi aggregometer (Chrono-Log Corp.) at 37°C with constant stirring of the platelet suspension at 1200 rpm. Aggregation was quantitated by monitoring increase in light transmittance through the stirred platelet suspension.

The $IC_{50}$'s (the concentration which inhibited aggregation by 50% as judged by the percent decrease in the initial slope of the aggregation curve) of the peptides were as follows:

| | |
|---|---|
| $(KR)_7$ | 3 micromolar |
| $(KR)_5$ | 50 micromolar |
| $(KR)_3$ | 250 millimolar |

At a concentration of 100 micromolar the following peptides inhibited aggregation to the following extent:

| | |
|---|---|
| $(KR)_7$ | 82% inhibition |
| $(KR)_5$ | 50% inhibition |
| $(KR)_3$ | no significant inhibition |
| $(R)_8GDV$ | 70% inhibition |

Inhibition of asialo-vWF-induced aggregation was determined using platelet-rich plasma prepared by differential centrifugation of blood drawn into 11 millimolar trisodium citrate anticoagulant. The platelet count was adjusted to $3 \times 10^{11}$/liter. The peptides, at a concentration of 55 micromolar, were incubated with platelet rich plasma for 5 minutes at 37°C. Asialo-vWF was then added at a final concentration of 15 micrograms/ml. Reaction mixtures were prepared in siliconized glass cuvettes and then placed in a Lumi aggregometer (Chrono-Log Corp.) at 37°C with constant stirring of the platelet suspension at 1200 rpm. Aggregation was quantitated by monitoring increase in light transmittance through the stirred platelet suspension.

Inhibition of aggregation by the peptides was as follows:

8

| (KR)7 | 100% inhibition |
| (KR)5 | 88% inhibition |
| (KR)3 | no significant inhibition |
| (R)8GDV | 92% inhibition |

**Claims**

1. A peptide of a 45 kDa amino terminal tryptic fragment of glycocalicin selected from the following amino acid sequence:

```
              10          20          30          40          50          60
               |           |           |           |           |           |
   HPICEVSKVASHLEVNCDKRNLTALPPDLPKDTTILHLSENLLYTFSLATLMPYTRLTQL

              70          80          90         100         110         120
               |           |           |           |           |           |
   NLDRCELTKLQVDGTLPVLGTLDLSHNQLQSLPLLGQTLPALTVLDVSFNRLTSLPLGAL

             130         140         150         160         170         180
               |           |           |           |           |           |
   RGLGELQELYLKGNELKTLPPGLLTPTPKLEKLSLANNNLTELPAGLLNGLENLDTLLLQ

             190         200         210         220         230         240
               |           |           |           |           |           |
   ENSLYTIPKGFFGSHLLPFAFLHGNPWLCNCEILYFRRWLQDNAENVYVWKQGVDVKAMT

             250         260         270         280         290
               |           |           |           |           |
   SNVASVQCDNSDKFPVYKYPGKGCPTLGDEGDTDLYDYYPEEDTEGDKVRATR
```

which inhibits binding of von Willebrand factor to platelet membrane glycoprotein Ib and platelet membrane glycoprotein Ib related molecules or other cells and cell matrices.

2. A sequential subset of a 45 kDa amino terminal tryptic fragment of glycocalicin selected from the following amino acid sequence:

```
         10        20        30        40        50        60
          |         |         |         |         |         |
HPICEVSKVASHLEVNCDKRNLTALPPDLPKDTTILHLSENLLYTFSLATLMPYTRLTQL

         70        80        90       100       110       120
          |         |         |         |         |         |
NLDRCELTKLQVDGTLPVLGTLDLSHNQLQSLPLLGQTLPALTVLDVSFNRLTSLPLGAL

        130       140       150       160       170       180
          |         |         |         |         |         |
RGLGELQELYLKGNELKTLPPGLLTPTPKLEKLSLANNNLTELPAGLLNGLENLDTLLLQ

        190       200       210       220       230       240
          |         |         |         |         |         |
ENSLYTIPKGFFGSHLLPFAFLHGNPWLCNCEILYFRRWLQDNAENVYVWKQGVDVKAMT

        250       260       270       280       290
          |         |         |         |         |
SNVASVQCDNSDKFPVYKYPGKGCPTLGDEGDTDLYDYYPEEDTEGDKVRATR
```

which inhibits binding of von Willebrand factor to platelet membrane glycoprotein Ib and platelet membrane glycoprotein Ib related molecules or other cells and cell matrices.

3. A peptide which inhibits binding of von Willebrand factor to platelet membrane glycoprotein Ib and platelet membrane glycoprotein Ib related molecules or other cells and cell matrices selected from the group of peptides consisting of:DKRNLTALPPDLPKDTT; NLTALPPDLPKDTTI; PPDLPKDTTILHLSE; PGLLTPTPKLEKLSL; KQGVDVKAMTSNVAS; GDTDLYDYYPEEDTE; EEDTEGDKVRATRTV; PPDLPKDTT; or EEDTE.

4. A peptide which comprises,within the peptide of Claim 3,any sequential subset of amino acids and derivatives thereof.

5. A peptide having the general formula $(KR)_n$, wherein $n=2-10$ and $R_n$ wherein $n=2-20$ and any derivatives thereof which inhibits binding of von Willebrand factor to platelet membrane glycoprotein Ib and platelet membrane glycoprotein Ib related molecules or other cells and cell matrices.

6. A method for inhibiting aggregation of platelets to each other comprising contacting the platelets with a peptide as claimed in any of claims 1-5 in an amount of said peptide effective to inhibit said aggregation.

7. A method for inhibiting thrombosis in a patient which comprises administering to said patient a peptide as claimed in any of claims 1-5 in an amount of said peptide effective to inhibit said thrombosis.